# EUROPEAN PATENT APPLICATION

(11) **EP 2 755 030 A1**
(43) Date of publication of application: **16.07.2014**
(21) Application number: 13151267.5
(22) Date of filing: 15.01.2013
(51) Int. Cl.: G01N 33/574, G01N 33/68

(54) **Prognostic marker for endometrial carcinoma**

(71) Applicant: Bergen Teknologioverføring AS, 5006 Bergen (NO)
(72) Inventor: Salvesen, Helga B., 5231 Paradis (NO); Trovik, Jone, 5019 Bergen (NO); Wik, Elisabeth, 5067 Bergen (NO); Akslen, Lars Andreas, 5252 Soreidgrend (NO)
(74) Representative: Kröncke, Rolf

(57) **Abstract**

The present invention relates to a method for diagnosing or determining the status of endometrial carcinoma in an individual afflicted with or suspected to be afflicted with endometrial carcinoma based on the phosphorylated stathmin compound whereby said stathmin is phosphorylated at position Serine 38. Moreover, the present invention relates to a method for evaluating the probability of survival, the clinical outcome or the treatment course of an individual afflicted with or suspected to be afflicted with endometrial carcinoma. In another aspect, the present invention relates to the stratification of the therapeutic regimen of an individual with endometrial carcinoma. In particular, the present invention provides a method for diagnosing or identifying endomentrial carcinoma with high grade aggressive phenotype, in particular, having low response and susceptibility to chemotherapeutic regimen, in particular, with respect to micro-tubule stabilization based regimen including treatment with taxanes. Moreover, the present invention relates to a kit for use in any of the above-referenced methods comprising means for determining the level or amount of phosphorylated pStathmin(S38) as well as assays for conducting the method according to the present invention. In one embodiment, the methods according to the present invention include the combined determination of stathmin and phosphorylated stathmin (pStathmin(S38)).

SEQUENCE LISTING

<110>  Bergen Teknologioverforing AS

<120>  Prognostic marker for endometrial cancer

<130>  4200-022-EP-1

<160>  2

<170>  PatentIn version 3.5

<210>  1
<211>  174
<212>  PRT
<213>  homo sapiens

<400>  1

Met Ala Ser Ser Asp Ile Gln Val Lys Glu Leu Glu Lys Arg Ala Ser
1               5                   10                  15

Gly Gln Ala Phe Glu Leu Ile Leu Ser Pro Arg Ser Lys Glu Ser Val
            20                  25                  30

Pro Glu Phe Pro Leu Ser Pro Pro Lys Lys Lys Asp Leu Ser Leu Glu
        35                  40                  45

Glu Ile Gln Lys Lys Leu Glu Ala Ala Glu Glu Arg Arg Lys Ser His
    50                  55                  60

Glu Ala Glu Val Leu Lys Gln Leu Ala Glu Lys Arg Glu His Glu Lys
65                  70                  75                  80

Glu Val Leu Gln Lys Ala Ile Glu Glu Asn Asn Asn Phe Ser Lys Met
                85                  90                  95

Ala Glu Glu Lys Leu Thr His Lys Met Glu Ala Asn Lys Glu Asn Arg
            100                 105                 110

Glu Ala Gln Met Ala Ala Lys Leu Glu Arg Leu Arg Glu Lys Met Tyr
        115                 120                 125

Phe Trp Thr His Gly Pro Gly Ala His Pro Ala Gln Ile Ser Ala Glu
    130                 135                 140

Gln Ser Cys Leu His Ser Val Pro Ala Leu Cys Pro Ala Leu Gly Leu
145                 150                 155                 160

Gln Ser Ala Leu Ile Thr Trp Ser Asp Leu Ser His His His
                165                 170

<210>  2
<211>  2265

<212>  DNA
<213>  homo sapiens

<400>  2
gctctcggcc aatgcggagc cccgcgcgga ggtcacgtgc ctctgtttgg cgcttttgtg      60

cgcgcccggg tctgttggtg ctcagagtgt ggtcaggcgg ctcggactga gcaggacttt     120

ccttatccca gttgattgtg cagaatacac tgcctgtcgc ttgtcttcta ttcaccatgg     180

cttcttctga tatccaggtg aaagaactgg agaagcgtgc ctcaggccag gcttttgagc     240

tgattctcag ccctcggtca aaagaatctg ttccagaatt ccccctttcc cctccaaaga     300

agaaggatct ttccctggag gaaattcaga agaaattaga agctgcagaa gaaagacgca     360

agtcccatga agctgaggtc ttgaagcagc tggctgagaa acgagagcac gagaaagaag     420

tgcttcagaa ggcaatagaa gagaacaaca acttcagtaa aatggcagaa gagaaactga     480

cccacaaaat ggaagctaat aaagagaacc gagaggcaca aatggctgcc aaactggaac     540

gtttgcgaga gaagatgtac ttctggactc acgggcctgg ggcccaccca gcacagatct     600

ctgctgagca atcttgtctc cactctgttc ctgccctttg cccagccctg ggcctccaat     660

ctgcattgat tacctggtct gatctctctc accatcacta ggtacttaat aaatatttgc     720

tgttgatgat agcaatgacc ttgagactga tgaacagtct ggccaagagg atccttgatg     780

tggaagatag aaaaagcctt tggggtcagg cagacttgga ttctaatacc agccagttct     840

gcttgctgtg tctgagcctc agtttactca tctgtgaaga ggaggtagca agaatgaaaa     900

tgcctgcctt gtggtttgtt gtaaggacag acactgccaa cgtagagggc ccagcagctc     960

acagaccagt tgctctgaga gcagaccact cttgccttga tggtagggaa ctatttttgt    1020

gcgtggcaag tgggacctta ggaaggaagg caactgtgag gcttctgaga aggaccctac    1080

acaagggagt ttcctcccag ggcaggtgaa tggagagggt ggcagaagcc tacggaaggg    1140

ggtcacaggg atcagctaga gagtgccacc acccttcctg gggaatgcag ggcaaggtcc    1200

ctggtgggag ttttcctggg aagccaaaga agcgcccaac aaagacagaa tcaacatttg    1260

ggtacctttg gtacccagag gcagcaatgc caactacaac cacactggaa gaagaagacc    1320

ttctccgcat agattctctg atctcttcct ccttcatggc accagccctg gggaaccagc    1380

atggtgggga aataatgaag ctggaataca accacttaca gacttcacaa cctcctcctg    1440

tagataccaa agggatttta ggatcacatt ttatttctca cctgagcaag aaaagctaca    1500

ggagcatctc aagcagaggg caggagtctc cagaggagtt caaggggctc tggcaagaaa    1560

aatcaagggg ctgtgttcaa gaactggctc ccttggtgat tgtattacga agcccatgtg    1620

tgctggatgc tgatgaaatt gctgccaaat gcctgtgcag ccttggcaag gccctttatt    1680

tctctgggtc tccatttctc tctctctttt tttttttttt tttttttgga ggcagagtct    1740

cactctgtcg cccaggctgg agggcagtgg cgtgatctcg gctcactgca agccccacct    1800

tctgagttca cgccattcta ctgcctcagc ctcccgagta gctgggacta caggcgccca    1860

ccaccacgcc cggcttattt tttgtatttt tagtagagac ggggtttcac cgcattagcc 1920

aagatggtct cgatctcctg acctcgtgat tcacccacct cagcctccca aagtgctggg 1980

attacaggca tgagccactg cgcccggcct gggtctccgt ttctctagct gtgaaatgac 2040

tgttctaaaa gagccctgcc ggactttggc agtctgtaag aagacctgag ttcttctctc 2100

agttccaagc aggaaaattg aacataccct gagcccagag cctgcaacaa actctgggca 2160

gcctcaggaa gtcaggcagt gaagtcggaa aaatgatctc ttctgtatag ggagaaaata 2220

aaagtttaaa aatttgtaaa aaaaaaaaaa agaaaaaaaa aaaaa 2265

## Description

The present invention relates to a method for diagnosing or determining the status of endometrial carcinoma in an individual afflicted with or suspected to be afflicted with endometrial carcinoma based on the phosphorylated stathmin compound whereby said stathmin is phosphorylated at position Ser38. Moreover, the present invention relates to a method for evaluating the probability of survival, the clinical outcome or the treatment course of an individual afflicted with or suspected to be afflicted with endometrial carcinoma. In another aspect, the present invention relates to the stratification of the therapeutic regimen of an individual with endometrial carcinoma. In particular, the present invention provides a method for diagnosing or identifying endometrial carcinoma with high grade aggressive phenotype, in particular, having low response and susceptibility to chemotherapeutic regimen, in particular, with respect to microtubule stabilization based regimen including treatment with taxanes. Moreover, the present invention relates to a kit for use in any of the above-referenced methods comprising means for determining the level or amount of phosphorylated Stathmin(S38) as well as assays for conducting the method according to the present invention. In one embodiment, the methods according to the present invention include the combined determination of stathmin and phosphorylated stathmin.

### Prior art

Stathmin is a cytosolic phospho-protein known to be over-expressed in several malignancies (Belletti B, Baldassarre G., Expert Opin Ther Targets, 2011 Nov. 15(11):1249-66). It is suggested to be a marker of PTEN loss and to play a role in tumor progression. Further, it is considered important in signal transduction and involved in biological processes such as cell cycle progression, apoptosis and cell migration. Microtubular destabilization by Stathmin is suggested to happen through promotion of microtubule catastrophe or by preventing tubulin incorporation in growing microtubules. Stathmin protein function is regulated at a post-translational level by different mechanisms, of which phosphorylation is the most studied. Thus, Stathmin has 4 Serine phospho sites (Ser16, - 25, -38 and -63), and phosphorylation is shown to inactivate Stathmin's destabilizing effect on microtubules (Gavet O, et al., J Cell Sci. 1998, Nov; 111 (Pt. 22): 3333-46). Expression of Stathmin and the regulation of protein activity by phosphorylation are important for cell division; inactivation of Stathmin by phosphorylation is critical for proper formation of microtubules and the mitotic spindle, and thereby for entry into mitosis, whereas Stathmin's de-stabilizing effects on microtubules are important to disassemble the mitotic spindle as the cells move through late stages of mitosis. During M-phase progression, Cyclin Dependent Kinases (CDKs) 1/2 phosphorylate Ser25 and Ser38 and precede phosphorylation of Ser16 and Ser63 by other kinases, allowing the mitotic spindle to be properly organized. Ser38 is suggested to be phosphorylated also by kinases belonging to the MAPK family as well as the PI3Kinase pathway.

Stathmin protein expression has recently been reported to be a prognostic marker in endometrial cancer (Trovik J, et al., Overxpression Clin. Cancer Rs. 2011 May 10, WO 2012/076650) as well as in breast and urothelial carcinomas (Baquero MT, Cancer, 2012, Feb. 22, Lin WC, Urology, 2009, Dec; 74(6):1264-9), and has also been suggested as a predictive marker for response to taxane treatment in cancer (Alli E et al., Cancer Res. 2002 Dec. 1; 62(23):6864-9).

With a 2-3% lifetime risk among women, endometrial cancer is the most common pelvic gynaecologic malignancy in industrialized countries, and the incidence is increasing (Amant F et al. (2005), Lancet, 366:491-505.). Approximately 75% of cases are diagnosed with the tumor confined to the uterine corpus, but 15% - 20% of these recur after primary surgery with limited respond to systemic therapy. In light of these recurrences, patients with localized endometrial cancer have 2 major needs: (1) adjuvant therapies that will reduce the recurrence rate, and (2) the ability to target these therapies to the patients most likely to recur. In addition, women with metastatic disease require effective systemic therapy.

These needs, for effective systemic therapies and reliable prognostic markers, have been only partly addressed. The most common basis for determining risk of recurrent disease has been the categorization of endometrial cancer into two subtypes. The majority are type I, associated with good prognosis, low stage and grade, and endometrioid histology. In contrast, type II cancers are characterized by high stage and grade, non-endometrioid histology, and poor prognosis. However, the prognostic value of this distinction is limited as up to 20% of type I cancers recur, while half of type II cancers do not (Salvesen et al Lancet Oncol 2012, 1318), doi: 10.1016/51470-2045(12) 70213-9.

Endometrial cancer is the most frequent gynaecological cancer in industrialised countries. Although the majority have a good prognosis, up to 20 % recurs. To date there are few markers available to predict response to treatment of metastatic endometrial cancer. Patients with tumors expressing estrogen- and progesterone receptors have the best response to antihormonal treatment. Still, more markers are needed to predict response to other therapy modalities in patients with metastatic endometrial cancer.

Thus, it is still desirable to provide improved biomarkers allowing differentiation of different types of endometrial cancer and diagnosing endometrial cancer patients in risk of recurrence.

Thus, the first object of the present invention is to provide methods for diagnosing or determining the status of endometrial carcinoma in an individual afflicted with or suspected to be afflicted with endometrial carcinoma. Further, the present invention relates to providing a method of evaluating the probability of survival, the clinical outcome or the treatment course of an individual afflicted with or suspected to be afflicted with endometrial carcinoma as well as methods for the stratification of the therapeutic regimen of an individual with endometrial carcinoma. In particular, it is desirable to provide a method for diagnosing or identifying endometrial carcinoma with high-grade aggressive phenotype, in particular, having low response and susceptibility to treatment, in particular, microtubule stabilizing agent treatment like treatment with taxanes.

### Summary of the present invention

In a first aspect, the present invention relates to a method for diagnosing or determining the status of endometrial carcinoma in an individual afflicted with or suspected to be afflicted with endometrial carcinoma. The method according to the present invention includes the steps of determining physically the level or amount of phosphorylated stathmin, phosphorylated at position Serine38 (S38) according to SEQ. ID No. 1, (in the following abbreviated with pStathmin(S38)) in a sample of an individual and determining or diagnosing the status of the individual based on the level or amount of pStathmin(S38) in said sample.

In another aspect, the present invention provides a method for evaluating the probability of survival, the clinical outcome or the treatment course of an individual afflicted with or suspected to be afflicted with endometrial carcinoma comprising the step of
a) determining the level or amount of pStathmin(S38) in at least one sample of said individual and
b) predicting the probability of survival, the clinical outcome or determining the treatment course based on the amount or level of pStathmin(S38) present in said sample.

In a further aspect, the present invention provides a method for the stratification of the therapeutic regimen of an individual with endometrial carcinoma comprising the step of
a) determining the level or amount of pStathmin(S38) in at least one sample of said individual, and
b) stratifying the therapeutic regimen based on the amount or level of pStathmin(S38) present in said sample.

In addition, the present invention relates to a method for diagnosing or identifying endometrial carcinoma with high grade aggressive phenotype, in particular, having low response and susceptibility to chemotherapeutic treatment, like treatment with taxanes comprising the steps of:
a) determining the level or amount of pStathmin(S38) in at least one sample of said individual and
b) diagnosing or identifying the phenotype and the responsibility and susceptibility to chemotherapeutic treatment based on the amount or level of pStathmin(S38) present in said sample.

In particular, the present invention provides a method for monitoring the progression of endometrial carcinoma or for determining the transition from low to high grade endometrial carcinoma in an individual afflicted with endometrial carcinoma comprising the steps of:
a) determining the level or amount of pStathmin(S38) in a sample of said individual at a first point in time;
b) determining the level or amount of pStathmin(S38) in a sample of said individual at a second point in time; and
c) comparing the level or amount of pStathmin(S38) determined in step a) to the level or amount determined in step b) or to a reference value whereby an increase in the level or the amount relative to a reference value or to the level or amount determined in step a) is indicative for a transition from low grade to high grade or for progression of endometrial carcinoma.

Moreover, the present invention relates to the use of a kit for diagnosing or determining the status of endometrial carcinoma, or for predicting the probability of survival, the clinical outcome or determining the treatment course in an individual afflicted or suspected to be afflicted with endometrial carcinoma, or for the stratification of the therapeutic regimen of an individual afflicted with endometrial carcinoma, or for monitoring the progression of endometrial carcinoma or for determining the transition from low grade to high grade endometrial carcinoma in an individual, said kit comprising means for determining the level or amount of pStathmin(S38) and, optionally, stathmin; and instructions how to use said test kit for a method according to the present invention.

Finally, the present invention provides an assay for conducting the method according to the present invention comprising the means for determining the level or amount of pStathmin(S38) and, optionally, stathmin.

### Brief description of the drawings

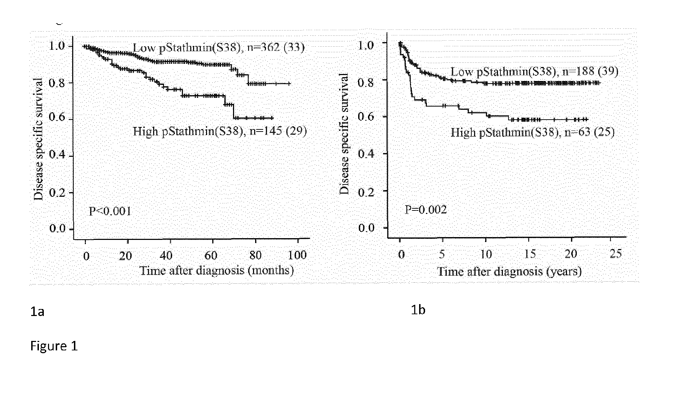

Figure 1. pStathmin(S38) IHC staining; high (A) and low (B) pStathmin(S38) levels. High level of pStathmin(S38) is significantly associated with poor disease specific survival in both the investigation(C) and validation cohorts (D).Survival curves are estimated by the Kaplan-Meier method. For each category the number of cases is given followed by the number of endometrial carcinoma deaths.

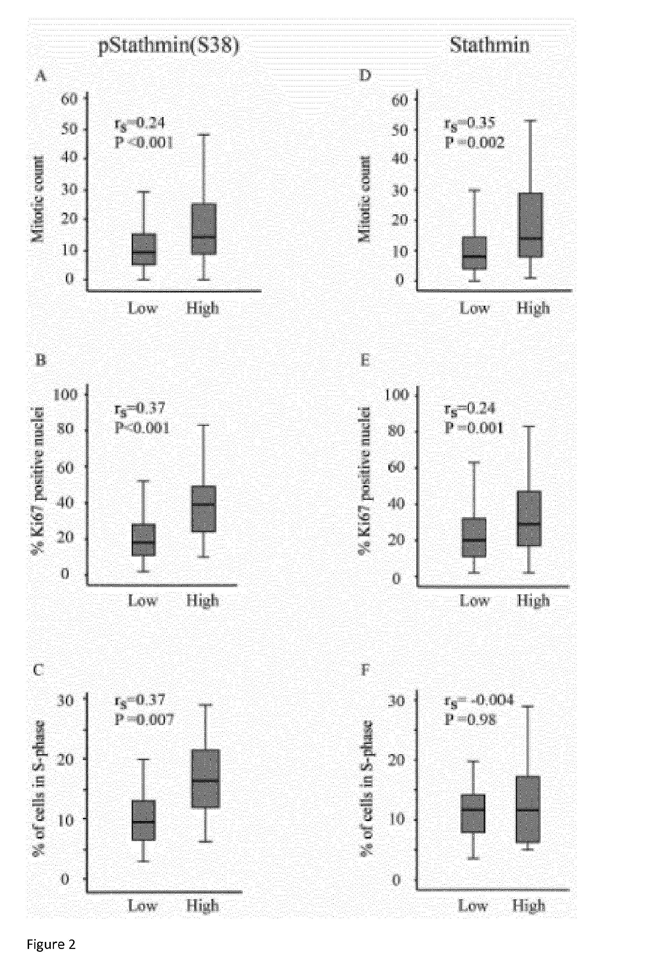

Figure 2. pStathmin(S38) identifies patients with different prognosis in subgroups of patients with expected good survival; Disease specific (A) and recurrence free (B) survival in endometrioid cases and in endometrioid, histologic grade 1 or 2 cases(C and D); and recurrence free survival (E) in FIGO I/II, endometrioid, histologic grade 1 or 2 tumors. Survival curves are estimated by the Kaplan-Meier method. For each category the number of cases is given followed by the number of endometrial carcinoma deaths.

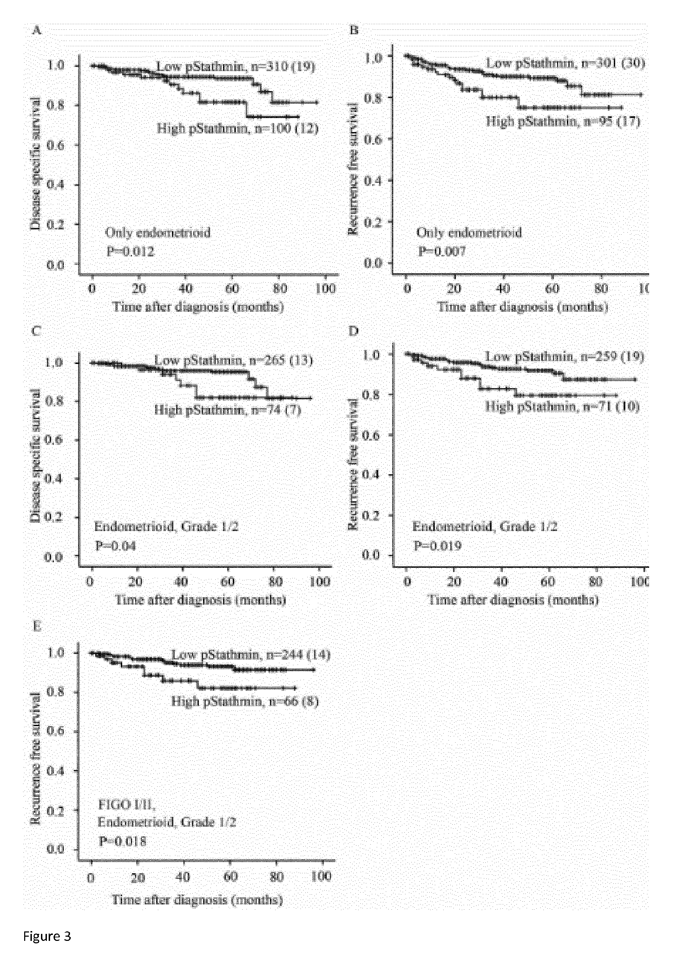

Figure 3. Correlation between levels of pStathmin(S38) and Stathmin in relation to markers for proliferative activity in primary tumors: mitotic counts per 10 high power field (x400), proportion of Ki67 positive tumor cells and assessment of S-phase fraction (validation series).r_{s =} Spearman's correlation coefficient.

### Detailed description of the present invention

In a first aspect, the present invention relates to a method for diagnosing or determining the status of endometrial carcinoma in an individual afflicted with or suspected to be afflicted with endometrial carcinoma, comprising:
a) determining the level or amount of phosphorylated stathmin1 phosphorylated Serine at position 38 according to SEQ ID No. 1 (pStathmin(S38)) in a sample of said individual and
b) determining or diagnosing status of the individual based on the level or amount of pStathmin(S38) in said sample.

In this connection, the term "determining" or "diagnosing" includes the step of physically determining or physically diagnosing the level or amount of pStathmin(S38). That is, the method includes the step of measuring the level or amount of pStathmin(S38) with known methods useful for measuring the level or amount and using said measured level or amounts to determine or to diagnose accordingly.

In another aspect, the present invention relates to a method for evaluating the probability of survival, the clinical outcome or the treatment course of an individual afflicted with or suspected to be afflicted with endometrial carcinoma comprising the step of
a) determining the level or amount of pStathmin(S38) in at least one sample of said individual and
b) predicting the probability of survival, the clinical outcome or determining the treatment course based on the amount or level of pStathmin(S38) present in said sample.

Moreover, a method is provided for the stratification of the therapeutic regimen of an individual with endometrial carcinoma comprising the step of
a) determining the level or amount of pStathmin(S38) in at least one sample of said individual, and
b) stratifying the therapeutic regimen based on the amount or level of pStathmin(S38) present in said sample.

Further, the present invention relates to a method for diagnosing or identifying endometrial carcinoma with high grade aggressive phenotype, in particular, having low response and susceptibility to chemotherapeutic treatment, like treatment with taxanes comprising the steps of:
a) determining the level or amount of pStathmin(S38) in at least one sample of said individual and
b) diagnosing or identifying the phenotype and the responsibility and susceptibility to chemotherapeutic treatment based on the amount or level of pStathmin(S38) present in said sample.

In addition, the present invention provides a method monitoring the progression of endometrial carcinoma or for determining the transition from low grad to high grade endometrial carcinoma in an individual afflicted with endometrial carcinoma comprising the steps of:
a) determining the level or amount of pStathmin(S38) in a sample of said individual at a first point in time;
b) determining the level or amount of pStathmin(S38) in a sample of said individual at a second point in time; and
c) comparing the level or amount of pStathmin(S38) determined in step a) to the level or amount determined in step b) or to a reference value whereby an increase in the level or the amount relative to a reference value or to the level or amount determined in step a) is indicative for a transition from low grade to high grade or for progression of endometrial carcinoma

As used herein, the term "comprise" or "comprising" as well as "contain" or "containing" includes the embodiments of "consist of" or "consisting of".

The pStathmin(S38) molecule is a stathmin being phosphorylated at position Ser38 according to SEQ. ID No. 1.

As used herein Stathmin without specifically identifying a phosphorylation refers to the Stathmin polypeptide or fragments thereof as well as to the STMN1 gene, genbank Acc. No. NM_001145454, in general without determining the phosphorylation of said polypeptide.

It has been recognized that apart from the Stathmin compound known to be an independent diagnostic marker for endometrial carcinoma, the phosphorylated stathmin S38 (pStathmin(S38)) being phosphporylated in position Ser38 is superior over stathmin as demonstrated in the examples. In particular, as demonstrated below in a multivariate survival analysis pSTMN1 maintained an independent prognostic while stathmin did not.

In one embodiment of the present invention, the method may further comprise the step of determining the level or amount of Stathmin in a sample of the individual to be tested. A combination of Stathmin and pStathmin(S38) according to the present invention can increase the validity or significance of the methods according to the present invention.

According to the present invention, the methods disclosed herein relates to *in vitro* and/or *in vivo* methods, respectively. Preferably, the methods are *in vitro* methods based on samples obtained from individuals and provided *in vitro.*

The method of the present invention allows to differentiate between high grade aggressive phenotype of endometrial carcinoma and low grade phenotype of endometrial cancer and, thus, allow to differentiate or determine the susceptibility or responsiveness of an individual in need of a treatment of endometrial cancer and other types of cancer if applicable, to chemotherapeutic drugs, in particular, drugs disrupting the microtubule function, namely microtubule stabilizing agents, in particular, taxanes.

As used herein, the term "taxanes" refers to diterpenes having cytostatic activity. Examples of suitable taxanes includes paclitaxel and docetaxel. A skilled person is well aware of suitable forms of taxanes including salts and solvents thereof.

Hence, the present invention relates to methods allowing differentiation of endometrial carcinoma, in particular, allowing to differentiate between low grade and high grade aggressive phenotype in endometrial carcinoma based on the pStathmin(S38) expression for determining the treatment regimen or the clinical outcome in an individual suffering therefrom. The present invention is directed to the prognosis as well as to the stratification of endometrial tumors and its therapy with respect to chemotherapeutic drugs, in particular, of metastatic endometrial carcinomas.

That is, in one aspect, the present invention relates to endometrial carcinoma and the importance of the P13K pathway in patients having aggressive endometrial cancer. The high pStathmin(S38) expression, e.g. detected by immunohistochemical methods, is associated with poor recurrence free survival and with poor recurrence free and overall survival in patients suffering from endometrial carcinomas. As demonstrated herein, high pStathmin(S38) expression represents an independent prognostic indicator allowing to differentiate between high grade aggressive phenotype and low grade phenotype endometrial carcinoma and the clinical outcome of the susceptibility and responsiveness of an individual need of a treatment of endometrial carcinoma to chemotherapeutic drugs, in particular, drugs disrupting the microtubule functions, like taxanes. In particular, high pStathmin(S38) expression is associated with poor prognosis in the otherwise low risk endometroid subgroup.

Moreover, given in multivariate studies pStathmin(S38) represents an independent prognostic marker while the previously described Stathmin expression does not.

The present invention covers the determination of pStathmin(S38) expression in methods allowing the differentiation of endometrial carcinoma, as well as stratification of endometrial tumors and its therapy as well as monitoring the chemotherapy. Furthermore, the present invention provides a method for evaluating the probability of survival as well as methods for providing a prognosis of a subject suffering from endometrial carcinoma, responding on chemotherapy based on pSTMN1 S38 expression.

In a preferred embodiment, the method for the stratification of the therapeutic regimen or the treatment course in an individual with endometrial carcinoma is intended to be treated or treated with chemotherapeutic drugs, like microtubule stabilizing agents, including taxanes.

In addition, the method according to the present invention comprises determining physically the expression status of pStathmin(S38). It has been recognized that high pStathmin(S38) expression is associated with pure recurrence free survival and overall survival in patients suffering from endometrial cancer. In particular, the pStathmin(S38) expression allows to differentiate between high grade aggressive phenotype and low grade aggressive phenotype of endometrial cancer whereby high pStathmin(S38) is associated with high grade aggressive phenotype of endometrial carcinoma, and, in addition, to determine the susceptibility or responsiveness of said individual in the need of a treatment of endometrial cancer to chemotherapeutic drugs, in particular, drugs disrupting the microtubule functions, like taxanes.

Moreover, the present invention relates to a method for monitoring the progression of endometrial carcinoma the progression of endometrial carcinoma or for determining the transition from low grad to high grade endometrial carcinoma in an individual afflicted with endometrial carcinoma comprising the steps of:
a) determining the level or amount of pStathmin(S38) in a sample of said individual at a first point in time;
b) determining the level or amount of pStathmin(S38) in a sample of said individual at a second point in time; and
c) comparing the level or amount of pStathmin(S38) determined in step a) to the level or amount determined in step b) or to a reference value whereby an increase in the level or the amount relative to a reference value or to the level or amount determined in step a) is indicative for a transition from low grade to high grade or for progression of endometrial carcinoma.

Further, the present invention relates to a method for the stratification of a chemotherapeutic therapy of endometrial carcinoma based on the pStathmin(S38) expression whereby high pStathmin(S38) expression identifies low responsiveness to microtubule stabilizing agents including taxanes.

The present inventors aimed in demonstrating that pStathmin(S38) expression predicts the response to chemotherapeutic drugs, in particular, low response to microtubule stabilizing agents including taxanes. Hence, pStathmin(S38) expression is useful as a marker for the treatment of metastatic endomentrial carcinoma but also in endometrial carcinoma in general.

The method may further comprise the step of determining the level or amount of Stathmin. While the pStathmin(S38) expression status is typically determined on amino acid level, the Stathmin expression status may be determined on nucleic acid or amino acid level.

Preferred embodiments include the detection on protein level by using appropriate antibodies and systems comprising the same. Suitable methods include ELISA, western blot, immunohistochemical or immunofluorescence detection.

The sample obtained from said individual is selected from tissue, body fluid, in particular, primary and metastatic malignant lesions.

According to a further embodiment of the invention, high level or amounts of pStathmin(S38), and, optionally, Stathmin, is indicative for high grade aggressive endomentrial carcinoma, lower probability of survival and worsened clinical outcome, lower response and susceptibility to chemotherapeutic treatment, in particular, with microtubule stabilizing agent including taxanes in said individual.

In another aspect, a kit for use in diagnosing or determining the status of endometrial carcinoma, or for predicting the probability of survival, the clinical outcome or determining the treatment course in an individual afflicted or suspected to be afflicted with endometrial carcinoma, or for the stratification of the therapeutic regimen of an individual afflicted with endometrial carcinoma, or for monitoring the progression of endometrial carcinoma or for determining the transition from low grade to high grade endometrial carcinoma in an individual, said kit comprising means for determining the level or amount of pStathmin(S38) and, optionally, Stathmin; and instructions how to use said test kit for a method according to the present invention is provided. Said kit is particularly useful for predicting the response to microtubule stabilizing agents including taxanes in an individual considering a therapeutic regimen when using said chemotherapeutic agent in said individual. In particular, in case of the treatment of endometrial cancer in general, and in particular, of metastatic endometrial cancer, the methods and kits according to the present invention are useful for stratifying the therapy thereof. For example, when microtubule stabilizing agents including taxanes are used for the treatment of metastatic cancer, like metastatic endometrial cancer, determining the pStathmin(S38) and, optionally, the Stathmin status allowed to stratify and to diagnose therapeutic success of treatment with said chemotherapeutic agent, accordingly.

That is, a few markers are available to predict response to treatment of metastatic endometrial cancer. Patients with tumors expressing estrogen and progesterone receptors have the best response to antihormonal treatment. However, more markers are needed to predict the response to other therapeutic modalities patients with endometrial cancer, in particular, metastatic endometrial cancer. It has been demonstrated herein that the level of pSTMN1S38 expression optionally in combination with the STMN1 expression, allows to predict response to tubule stabilizing chemotherapy in endometrial cancer. A typical example of tubule stabilizing therapy includes taxol, taxotere, eleutherobin, Sarcodicytin A, Sarcodicytin B, Epothilone A, Epothilone B, Discodermolide, Laulimanide, Isolaulimalide, Ixabepilone, Vinblastin, Vinkrestin, vinorelbin.

Finally, the present invention provides a method according to the present invention allowing for differentiation of complex hyperplasia with atypical or endometrial cancer.

In the following, the present invention will be further outlined by examples without limiting thereto.

### Examples

### MATERIAL AND METHODS

### Patients and tumor samples

Formalin fixed and paraffin embedded (FFPE) as well as fresh frozen endometrial carcinoma tumor specimens were retrieved from the Bergen Gynecologic Cancer Biobank, Norway, and related to clinical and histopathologic data in two independent series: I) The primary investigation set of fresh frozen and FFPE tumor tissue in parallel(n=122/518, respectively), prospectively collected from May 2001 to December 2010; II)The retrospectively collected population based validation series consisting of FFPE tumor tissue from 286patientsdiagnosed from 1980-1990 (Salvesen HB, Clin Cancer Res. 2000 Jan. 6(1):153-9). All patients were treated at the Section for Gynecological Cancer, Haukeland University Hospital, a referral hospital for patients in the Western Health Region of Norway including Hordaland County, Bergen, Norway, as previously reported (Trovik J et al., Gynecol Oncol. 2012 May; 125 (2):381-7).

### Primary investigation series (n=518)

The patients were prospectively enrolled in the period 2001-2010. Clinico-pathologic data including age at diagnosis, International Federation of Gynecology and Obstetrics (FIGO) stage according to the 2009 criteria, histologic type and grade were obtained from the clinical records and routine histopathology reports.

Median follow-up for survivors was 3.9 years (range 0.1-8). Patients were followed from the date of primary surgery until June 15^{th} 2011 or until death. The surgical treatment protocol was abdominal hysterectomy with bilateral salpingo-oophorectomy as primary treatment. The primary surgery also included pelvic lymphadenectomy as staging procedure for the majority of patients (78%). Adjuvant therapy was recommended for patients with FIGO stage ≥ II and high risk FIGO stage Ipatients, defined as non-endometrioid tumors or deeply infiltrating endometrioid grade 3 tumors.

### Validation series (n=286)

The patients were diagnosed with primary endometrial cancer in the period 1981-90.Clinico-pathologic data, retrospectively obtained, included age at diagnosis, FIGO stage according to the 1988 criteria, and histologic type and grade, based on the results from histopathologic revision. Median follow-up period for the survivors was 18.5 years (range 13.2-23.2). Last date of follow up was June 30^{th} 2004. The treatment protocol for this period was abdominal hysterectomy with bilateral salpingo-oophorectomy as primary treatment. Radiation and hormonal therapy were given as adjuvant therapy respectively to 180 (63%) and 7 (2%) of the patients.

Tumor specimens were investigated for levels of pStathmin(S38) and Stathmin by immunohistochemistry (IHC). Assessment of Ki67, mitotic count, and S-phase fraction in the validation series has previously been described (Salvesen HB et al., Clin Cancer Res. 1998 Nov; 4(11):2779-85). mRNA expression was assessed by DNA oligonucleotide microarray for a subset of 122 freshly frozen tumor specimens from the primary investigation series.

### Tissue microarray (TMA)

Hematoxylin and eosin (H&E) stained slides from tumors were evaluated to identify areas with high tumor purity for retrieval of three 0.6 mm tissue cylinders to be mounted in a recipient paraffin block, using a custom-made precision instrument (Beecher Instruments, Silver Spring, MD, USA). TMA sections of 5µm were subsequently dewaxed with xylene/ethanol for immunohistochemical staining.

### Immunohistochemistry

Details on the Stathmin staining are previously reported (Trovik et al., Clin Cancer Res. 2011 May 10). For pStathmin(S38), staining procedures were performed using the Leica Microsystems Bond III Autostainer automated slide processing equipment. Heat Induced Epitope Retrieval was applied for ten minutes in Bond Epitope Retrieval Solution 1 (citrate buffer and surfactant, pH5.6-6.1, Leica Biosystems, IL, USA). Sections were blocked for peroxidase activity(Bond Refine Block, Leica Biosystems)and incubated during fifteen minutes at room temperature with a rabbit monoclonal phospho-Stathmin antibody (clone D19H10, Cell Signaling Technologies, catalog #4191) diluted 1 :200 in Bond Primary Diluent. The Bond Polymer Refine detection (Leica Biosystems) was added for ten minutes at room temperature for antibody detection. Finally, slides were briefly counterstained with Leica SurgiPathSelecTech Hematoxylin (Leica Biosystems) for five minutes. Samples of normal tonsils known to yield positive staining for pStathmin(S38) were used as positive controls and by substituting the primary antibody with diluent only, as negative control.

### Evaluation of staining

Blinded for patient characteristics and outcome, the slides were evaluated by two of the authors (E.W. and H.S.), using a standard light microscope. A semi-quantitative grading system incorporating staining intensity (score 0-3) and area of tumor with positive staining (0= no staining, 1=<10%, 2=10-50% and 3=>50% of tumor cells) was applied. Staining index (SI) was calculated as the product of staining intensity and are ranging from 0-9. A cut-off representing the upper quartile (SI>4) was used to define high level of pStathmin(S38).For Stathmin, the upper quartile (SI=9) defined high immunohistochemical expression, as previously reported (Trovik J et al., Clin Cancer Res. 2011 May 10).

### Fluorescence-in-situ-hybridization (FISH)

PIK3CA copy number alterations were assessed by fluorescent *in situ* hybridization (FISH) for 66 cases: The area of highest tumor grade was identified on H&E-stained slides. Tissue microarrays were prepared as reported above and TMA sections were treated at 56C° overnight before deparaffinization. Paraffin Pretreatment of TMA sections was performed according to the Reagent Kit protocol (Vysis) before hybridization. Dual color FISH was performed by using a digoxigenated BAC probe (BAC RP11-245C23, German Science Centre for Genome Research, DE) harboring the PIK3CA gene and a commercially available Spectrum-Orange labeled chromosome 3 centromeric probe (CEP3, D3Z1, Abbott) as a reference. Hybridization and posthybridization washes were done according to the 'LSI procedure' (Abbott, IL, USA). Visualization of the gene probe was carried out by using fluorescent isothiocyanate (FITC)-conjugated sheep anti-digoxigenin (Roche Diagnostics, Rotkreuz, Switzerland). Slides were counterstained with 125 ng/ml 4',6-diamino-2-phenylindole in an antifade solution. Copy numbers of gene specific and centromere signals were estimated for each tissue spot. A tumor was considered to have increased PIK3CA copy number if individual tumor cells on average had ≥ 3 gene signals, regardless of the gene/CEP signal copy number ratio.

### SNP array analysis and DNA sequencing

In the primary investigation series, genomic DNA was extracted from surgically dissected, fresh frozen primary tumors. SNP arrays interrogating 116 204 SNP loci were evaluated for 70 cases and Sanger sequencing of PIK3CA exon 9 and exon 20 for 245 cases.

### Oligonucleotide DNA microarray analyses

Extracted RNA was hybridized to Agilent Whole Human Genome Microarrays 44k (Cat.no. G4112F) according to the manufacturer's instructions *(www.agilent.com).* Arrays were scanned using the Agilent Microarray Scanner Bundle. Microarray signal intensities were determined using J-Express *(www.molmine.no).* Median spot signal data were used as intensity measure. The expression data were quantile normalized. False discovery rate (FDR) <0.1 was used as cut-off when identifying genes and pathways significantly differentially expressed between tumors with high versus low pStathmin(S38); using, respectively,Significance Analysis of Microarrays (SAM) for single genes detection and Gene set enrichment analysis (GSEA), based on gene sets available through MSigDB (www.broadinstitute.org/gsea/msigdb).

### PI3K activation score

A PI3K activation score was calculated in the DNA microarray data, based on a published PI3K signature (cell lines stably transfected with activated PIK3CA) (Gustfason et al, Sci Transl Med. 2010 April 7; 2(26):26ra5), subtracting the sum of the expression values of genes down-regulated from genes up-regulated in the transfected cell lines. Expression values of each gene were normalized by a common mean and scaled to the same standard deviation.

### Connectivity Map

The correlation between the global expression pattern and potential new therapeutics for patients with high tumor pStathmin(S38) was assessed in the primary investigation cohort. Associations between the pStathmin(S38) transcription signature and drug signatures in the Connectivity Map database (Lamb et al., Science 2006, Sept. 29; 313(5795):1919-35) were explored. Genes differentially expressed (FDR <0.1) between tumor subsets of low and high pStathmin(S38) levels were included in the signature as the basis for the analyses in Connectivity Map.

### Statistical analyses

Data were analyzed using SPSS (Statistical Package of Social Sciences), version 15.0 (SPSS, Inc., Chicago, IL). Probability of <0.05 was considered statistically significant, except for the DNA microarray analyses. Mann-Whitney U test and the Spearman's rank correlation was used for analyses of continuous variables between categories. Univariate survival analyses of time to death due to endometrial carcinoma (disease specific survival) and time to recurrence for patients without metastases at time of diagnosis (recurrence free survival) were performed using the Kaplan-Meier method. Entry date was the date of primary surgery. Patients who died from other causes were censored at the date of death. Differences in survival between groups were estimated by two sided logrank (Mantel Cox) tests. Categories were compared using Pearson's chi-square or Fisher's exact test when appropriate. Cox' proportional hazards method was used for multivariate survival analyses. Categorizing continuous variables, cut off points were based on median or quartile values, also considering the frequency distribution plot for each marker. Groups with similar survival were merged. Estimation of sample size was done by chi-square test using software East4, 2005 Cytel Software Corp. To reach 90% power detecting a 30% difference in 5-year survival (90% for patients with markers within normal range versus 60% with pathological markers) at a 5% level of significance, at least 65 patients were needed, assuming a ratio of 1:3 for positive versus negative markers.

### RESULTS

### pStathmin(S38) expression associates with clinico-pathologic phenotype and patient survival

pStathmin(S38) IHC staining was mainly cytoplasmic. High level of pStathmin(S38) was significantly associated with features of aggressive tumors, such as non-endometrioid histology, high histologic grade and high FIGO stage,as well as with recurrent disease (table 1).

**Table 1.Correlation between pStathmin(S38), clinico-pathologic phenotype and Stathmin expression in endometrial carcinomas.**

| | | **pStathmin(S38)^{a}** Primaryinvestigation series | | |
|---|---|---|---|---|
| Variables | Categories | Low n (%) | High n (%) | P^{b} |
| Patient age | < 65 | 187 (74) | 65 (26) | 0.15 |
| | ≥ 65 | 182 (68) | 84 (32) | |
| | | | | |
| Histologicsubtype | Endometrioid | 316 (75) | 104 (25) | <0.001 |
| | Non-endometrioid | 53 (54) | 45 (46) | |
| | | | | |
| Histologicgrade | Grade 1/2 | 272 (78) | 77 (22) | <0.001 |
| | Grade 3 | 94 (57) | 71 (43) | |
| | | | | |
| FIGO stage (2009) | I / II | 322 (75) | 110 (25) | <0.001 |
| | III / IV | 47 (56) | 39 (44) | |
| | | | | |
| Recurrence^{c} | No | 305 (75) | 101 (25) | 0.002 |
| | Yes | 46 (58) | 33 (42) | |
| | | | | |
| Stathmin^{d} | Low | 304 (81) | 71 (19) | <0.001 |
| | High | 26 (33) | 53 (67) | |

### Missing cases: Grade (n=4), Stathmin (n=64)

^{a}High pStathmin(S38) defined by score index >4. ^{b}Chi square test.^{c}Including only patients considered tumor free after primary surgery (n=485). ^{d}High Stathmin defined by score index 9.

High pStathmin(S38) was also associated with shorter disease specific survival in both patient series studied (figure 1A and B). In the subsets of presumed low risk endometrioid cases only, grade 1 or 2 endometrioid tumors, and grade 1 or 2 endometrioidtumors in FIGO I/II cases, high pStathmin(S38) was still associated with significantly worse outcome compared to low pStathmin(S38) (figure 2A-E).

In multivariate survival analyses, high pStathmin(S38) independently predicted poor prognosis adjusted for histologic subtype, histologic grade and myometrial infiltration among patients with tumors confined to the uterus (FIGO stage I/II) (table 2).

**Table 2. Cox's proportional hazard regression model used to estimate the prognostic value of pStathmin(S38) in endometrial carcinomas confined to the uterus,in relation to histopathologic variables.**

| Variable | | N (%) | Unadjusted HR^{a} | 95% CI | P^{b} | Adjusted HR | 95% CI | P^{b} |
|---|---|---|---|---|---|---|---|---|
| Histologic subtype | | | | 4.1-20.8 | <0.001 | | 1.01-12.1 | 0.048 |
| | Endometrioid | 355 (85) | 1 | | | 1 | | |
| | Non-endometrioid | 61 (15) | 9.3 | | | 3.5 | | |
| Histologic grade | | | | 2.9-16.1 | <0.001 | | 0.7-8.7 | 0.2 |
| | Grade 1/2 | 308 (74) | 1 | | | 1 | | |
| | Grade 3 | 108 (26) | 6.9 | | | 2.4 | | |
| Myometrial infiltration | | | | 1.7-8.9 | 0.001 | | 1.6-8.2 | 0.003 |
| < 50% | | 300 (72) | 1 | | | 1 | | |
| ≥ 50% | | 116 (28) | 3.9 | | | 3.6 | | |
| pStathmin(S38) | | | | 1.4-7.1 | 0.005 | | 1.1-5.7 | 0.035 |
| | Low | 310 (75) | 1 | | | 1 | | |
| | High | 106 (25) | 3.2 | | | 2.5 | | |

| | | | | | | | | |
|---|---|---|---|---|---|---|---|---|
| N: number of cases; HR: Hazard ratio; CI: confidence interval;FIGO: International Federation of Gynecology and Obstetrics ^{a}Unadjusted HRsgiven for analyses of 399cases with data available for all variables in the multivariate analysis;^{b}Likelihood ratio test | | | | | | | | |

This pattern of prognostic impact of high pStathmin(S38) expression was also seen in the validation series (HR 2.2, 95% CI: 0.96-4.9, p=0.07)when adjusted for the same histopathologic variables.

### pStathmin(S38)expression adds prognostic information to Stathmin.

pStathmin(S38) was significantly correlated to Stathmin expression (table 1). As both pStathmin(S38) and Stathmin were shown to be independent prognostic markers in separate models, we further examined how pStathmin(S38) performed as a prognosticator compared to Stathmin. In a multivariate survival analysis, including both Stathmin and pStathmin(S38) expression and adjusting for histologic subtype, histologic grade and myometrial infiltration, pStathmin(S38) maintained independent prognostic impact (HR 1.8, 95% CI 1.0-3.1, p=0.05), while Stathmin did not (table 3).

**Table 3. Primary investigation series. Cox's proportional hazard regression model used to estimate the prognostic value of pStathmin(S38) in endometrial carcinomas,in relation to histopathologic variables and Stathmin.**

| Variable | | N (%) | Unadjusted HR^{a} | 95% CI | P^{b} | Adjusted HR | 95% CI | P^{b} |
|---|---|---|---|---|---|---|---|---|
| Histologic sub-type | | | | 3.4-9.5 | <0.001 | | 1.3-4.9 | 0.009 |
| Endometrioid | | 366 (82) | 1 | | | 1 | | |
| Non-endometrioid | | 82 (18) | 5.7 | | | 2.5 | | |
| Histologic grade | | | | 3.3-9.5 | <0.001 | | 1.1-4.7 | 0.03 |
| | Grade 1/2 | 308 (69) | 1 | | | 1 | | |
| | Grade 3 | 82 818) | 5.6 | | | 2.3 | | |
| Myometrial infiltration | | | | 3.7-12.3 | <0.001 | | 3.2-10.8 | <0.00 1 |
| | < 50% | 284 (63) | 1 | | | 1 | | |
| | ≥ 50% | 164 (37) | 6.8 | | | 5.9 | | |
| Stathmin | | | | | | | 0.7-2.4 | 0.4 |
| | Low | 371 (83) | 1 | 1.4-4.1 | 0.002 | 1 | | |
| | High | 77 (17) | 2.4 | | | 1.3 | | |
| pStathmin(S38) | | | | 1.6-4.4 | <0.001 | | 1.0-3.1 | 0.05 |
| | Low | 326 (73) | 1 | | | 1 | | |
| | High | 122 (27) | 2.6 | | | 1.8 | | |

| | | | | | | | | |
|---|---|---|---|---|---|---|---|---|
| N: number of cases; HR: Hazard ratio; CI: confidence interval;FIGO: International Federation of Gynecology and Obstetrics ^{a}Unadjusted HRsgiven for analyses of 448cases with data available for all variables in the multivariate analysis;^{b}Likelihood ratio test | | | | | | | | |

There was no significant interaction between Stathmin and pStathmin(S38) in this survival model (p=0.2).Also in the validation series, pStathmin(S38) was superior to Stathmin, adjusting for the same histopathologic variables (table 4).

**Table 4. Validations series. Cox's proportional hazard regression model used to estimate the prognostic value of pStathmin(S38) in endometrial carcinomas,in relation to histopathologic variables and Stathmin.**

| Variable | | N (%) | Unadjusted HR^{a} | 95% CI | P^{b} | Adjusted HR | 95% CI | P^{b} |
|---|---|---|---|---|---|---|---|---|
| Histologic subtype | | | | 2.6-10.2 | <0.001 | | 1.5-11.1 | 0.00 5 |
| Endometrioid | | 169 (90) | 1 | | | 1 | | |
| Non-endometrioid | | 19 (10) | 5.1 | | | 4.1 | | |
| Histologic grade | | | | 1.6-5.3 | 0.001 | | 0.4-2.2 | 0.8 |
| | Grade 1/2 | 146 (78) | 1 | | | 1 | | |
| | Grade 3 | 42 (22) | 2.9 | | | 0.9 | | |
| Myometrial infiltration | | | | 2.6-9.8 | <0.001 | | 2.4-9.5 | <0.0 01 |
| < 50% | | 112 (60) | 1 | | | 1 | | |
| ≥ 50% | | 76 (40) | 5 | | | 4.8 | | |
| Stathmin | | | | | | | 0.9-3.4 | 0.1 |
| | Low | 128 (68) | 1 | 1.7-5.6 | <0.001 | 1 | | |
| | High | 60 (32) | 3.1 | | | 1.7 | | |
| pStathmin(S38) | | | | 1.5-4.9 | 0.001 | | 1.0-3.7 | 0.05 |
| | Low | 140 (74) | 1 | | | 1 | | |
| | High | 48 (26) | 2.7 | | | 1.9 | | |

| | | | | | | | | |
|---|---|---|---|---|---|---|---|---|
| N: number of cases; HR: Hazard ratio; CI: confidence interval;FIGO: International Federation of Gynecology and Obstetrics ^{a}Unadjusted HRsgiven for analyses of 188cases with data available for all variables in the multivariate analysis;^{b}Likelihood ratio test | | | | | | | | |

In the complete validation series, pStathmin(S38) had independent prognostic impact also when adjusting for age, histologic type, histologic grade, FIGO stage and Stathmin (table 5).

**Table 5. Validations series. Cox's proportional hazard regression model used to estimate the prognostic value of pStathmin(S38) in endometrial carcinomas,in relation to standard clinico-pathologic variables and Stathmin.**

| Variable | | N (%) | Unadjusted HR^{a} | 95% CI | P^{b} | Adjusted HR | 95% CI | P^{b} |
|---|---|---|---|---|---|---|---|---|
| Age at diagnosis^{d} | | 221 | 1.05 | 1.03-1.1 | <0.001 | 1.06 | 1.03-1.1 | <0.00 1 |
| Histologic subtype | | | | 2.6-8.5 | <0.001 | | 0.9-4.6 | 0.09 |
| Endometrioid | | 197 (89) | 1 | | | 1 | | |
| Non-endometrioid | | 24 (11) | 4.7 | | | 2 | | |
| Histologic grade | | | | 1.8-5.1 | <0.001 | | 0.5-2.6 | 0.7 |
| | Grade 1/2 | 169 (76) | 1 | | | 1 | | |
| | Grade 3 | 52 (24) | 3 | | | 1.2 | | |
| FIGO stage | | | | 5.6-16.3 | <0.001 | | 5.7-17.6 | <0.00 1 |
| | I/II | 180 (81) | 1 | | | 1 | | |
| | III/IV | 41 (19) | 9.6 | | | 10.1 | | |
| Stathmin | | | | | | | 0.8-2.4 | 0.2 |
| | Low | 157 (71) | 1 | 1.2-3.5 | 0.007 | 1 | | |
| | High | 64 (29) | 2.1 | | | 1.4 | | |
| pStathmin(S38) | | | | 1.4-3.9 | 0.002 | | 1.03-3.2 | 0.04 |
| | Low | 163 (74) | 1 | | | 1 | | |
| | High | 58 (26) | 2.3 | | | 1.8 | | |

| | | | | | | | | |
|---|---|---|---|---|---|---|---|---|
| N: number of cases; HR: Hazard ratio; CI: confidence interval;FIGO: International Federation of Gynecology and Obstetrics ^{a}Unadjusted HRsgiven for analyses of 221cases with data available for all variables in the multivariate analysis;^{b}Likelihood ratio test; ^{d}continuous variable | | | | | | | | |

### Integrated analyses associate high pStathmin(S38) expression to tumor cell proliferation.

Transcriptional differences between tumors with high versus low levels of pStathmin(S38) were explored by pathway analyses (GSEA) of DNA microarray data (primary investigation series). Gene sets comprising genes involved in cell cycle progression and cell proliferation were highly enriched in tumors with high pStathmin(S38) by IHC. These results, together with known functions of Stathmin and phosphorylation of the protein in relation to mitosis, support that pStathmin(S38) might be important for tumor cell proliferation in endometrial carcinomas. To further examine this hypothesis, we assessed the correlation between pStathmin(S38) and a panel of measures for cell prolife ratio n such as mitotic count, percentage of Ki67 positive tumor nuclei and S-phase fraction by flow cytometry (validation series) has been assessed. Consistently, high pStathmin(S38)was significantly correlated with high proliferation assessed by all these methods (figure 3A-C).Also, high Stathmin protein expression was correlated with similar strength to high mitotic count and proportion of Ki67 positive tumor cells, but not to S-phase fraction (figure 3D-F).

In sum, the data support that pstathmin (S38) is related to tumor cell proliferation and adds important and clinically relevant prognostic information in endometrial cancer patients, also among presumed low risk cases.

### PI3K/mTORare suggested as potential targetsin tumors with high pStathmin(S38)

Connectivity Map version 02 was queried for compounds negatively correlated to high pStathmin(S38) expression in endometrial carcinomas. The gene list acquired from class comparison analysis based on pStathmin(S38) IHC expressionstatus was used. Amongst 1309 small molecules represented in Connectivity Map, inhibitors of PI3K/mTOR and HSP90were the top ranked therapeutics identified as potential drugs to patients with high tumor pStathmin(S38), as listed in table 6.

**Table 6. Top ranked potential drugs and targets for therapy among endometrial cancer with high pStathmin(S38), based on Connectivity Map.**

| **Rank** | **Name of compound** | **Known target/action** | **N** | **P** |
|---|---|---|---|---|
| 1 | tanespimycin | HSP90 inhibitor | 62 | <0.00001 |
| 2 | sirolimus | mTOR inhibitor | 44 | <0.00001 |
| 3 | LY-294002 | PI3K inhibitor | 61 | <0.00001 |
| 4 | quinostatin | PI3K inhibitor | 2 | 0.0001 |
| 5 | thioridazine | Adrenerg and dopamine blocker | 20 | 0.0001 |
| 6 | geldanamycin | HSP90 inhibitor | 15 | 0.0007 |
| 7 | luteolin | Flavonoid; anti-proliferative properties | 4 | 0.0007 |
| 8 | apigenin | Flavonoid; anti-proliferative properties | 4 | 0.001 |
| 9 | vorinostat | HDAC inhibitor | 12 | 0.001 |
| 10 | camptothecin | Topoisomerase I inhibitor | 3 | 0.003 |

| | | | | |
|---|---|---|---|---|
| N = number of instances in which the compounds were tested in the Connectivity map | | | | |

The expression changes from the compounds tested were scored according to the pStathmin(S38) level signature. The p-value for each compound represents the distribution of this score in the N instances, compared with the distribution of these scores amongst all compounds tested, using a permutation test (Lamb, Science 2006)*.* The correlation between PI3Kinase pathway alterations and pStathmin(S38) expression in the tumors have been explored. High pStathmin(S38) associated significantly with amplification of the 3q26 region harboring PIK3CA as estimated by SNP array, increased absolute copy number of PIK3CA, estimated by FISH, and a high PI3K activation score, although not with PIK3CA mutations, neither with any significant association when investigating the correlation with exon 9 and exon 20 separately (p=0.7 and p=0.2, respectively). Taken together, high pStathmin(S38) expression associates with several potential measures of PI3K signaling activity which further underlines the potential for drugs inhibiting the PI3K signaling pathway in pStathmin(S38)-high endometrial carcinoma.

### DISCUSSION

Stathmin is shown to be a prognostic marker in various cancer types, such as breast and endometrial cancer, and is recently reported to predict lymph node metastases in a large multicenter study of endometrial cancer (Trovik J. et al., Clin Cancer Res. 2011 May 10). In contrast, the prognostic impact and possible clinical utility of phosphorylated Stathmin has not been much studied in human cancers, although the impact of phosphorylation at different Stathmin phospho sites has been explored in some experimental models, mainly in relation to the effects on microtubule formation, proliferation, cell migration and cancer invasion. In this study of endometrial cancer, pStathmin(S38) was strongly associated with different markers of tumor proliferation and showed a significant and independent association with patient survival above the information given by standard clinico-pathologic features and by Stathmin expression. A prognostic impact of pStathmin(S38) has not been previously shown for any cancer type. The findings have been validated in an independent patient cohort and indicate that pStathmin(S38) might be of practical use in the management of endometrial carcinoma patients, also in regard to identifying patients with higher risk for recurrent disease amongst presumed low risk cases. Moreover, transcriptional profiling indicated a relationship between pStathmin(S38) levels and inhibitors of PI3K and mTOR signaling, pointing to possible treatment effects of such drugs in pStathmin(S38)-high cases in particular. Thus, our findings strongly advocate the inclusion of pStathmin(S38) as a biomarker in relevant clinical trials of advanced endometrial cancers, studying the potential predictive value of this marker.

Both pStathmin(S38) and Stathmin IHC expression were strongly associated with the different proliferation markers investigated, possibly reflecting that both phosphorylated and unphosphorylated Stathmin might contribute to mitotic progression by microtubular stabilization and destabilizeation during the various phases of cell division (Belletti B, et al., Expert Opin Ther Targets, 2011 Nov. 15(11):1249-66). In line with this, gene sets related to cell cycle progression and proliferation to be particularly enriched among pStathmin(S38)-high tumors are described. A link between pStathmin(S38) and tumor cell proliferation is not previously reported in human cancer, although one study indicated that strong Stathmin expression was associated with higher Ki67 levels in regenerating liver tissue.

Stathmin has previously been associated with several potential measures for PI3K activation, such as PTEN loss, high levels of a transcriptional PI3K signature, and amplification of the 3q26 region, harboring PIK3CA, although not with PIK3CA mutations. Here, we found a similar pattern for pStathmin(S38), and in addition an association between high pStathmin(S38) and increased absolute PIK3CA copy number by FISH. Based on drug signatures, we found several compounds relevant for targeting the PI3Kinase pathway related to high pStathmin(S38)expression. The top ranked HSP90 inhibitors are shown to be crucial for functional folding of various proteins in multiple pathways, including AKT in the PI3K pathway. Also,HSP90 and PI3K inhibitors in combination are more effective than single drugs in cell line studies of various cancer types, and a clinical trial in advanced gastric cancer with combined HSP90/PI3K inhibition has been initiated (www.clinicaltrials.gov August 2012: NCT01613950).

## Claims

1. A method for diagnosing or determining the status of endometrial carcinoma in an individual afflicted with or suspected to be afflicted with endometrial carcinoma, comprising:
a) determining the level or amount of phosphorylated stathmin1 phosphorylated at Ser position 38 according to SEQ ID No. 1 (pStathmin(S38)) in a sample of said individual and
b) determining or diagnosing status of the individual based on the level or amount of pStathmin(S38) in said sample.

2. A method for evaluating the probability of survival, the clinical outcome or the treatment course of an individual afflicted with or suspected to be afflicted with endometrial carcinoma comprising the step of
a) determining the level or amount of pStathmin(S38) in at least one sample of said individual and
b) predicting the probability of survival, the clinical outcome or determining the treatment course based on the amount or level of pStathmin(S38) present in said sample.

3. A method for the stratification of the therapeutic regimen of an individual with endometrial carcinoma comprising the step of
a) determining the level or amount of pStathmin(S38) in at least one sample of said individual, and
b) stratifying the therapeutic regimen based on the amount or level of pStathmin(S38) present in said sample.

4. The method for the stratification of the therapeutic regimen or the treatment course according to claim 2 or 3 in an individual with endometrial carcinoma intended to be treated or treated with chemotherapy drugs, in particular, microtubule stabilizing agents including taxanes.

5. A method for diagnosing or identifying endometrial carcinoma with high grade aggressive phenotype, in particular, having low response and susceptibility to chemotherapeutic treatment, like treatment with microtubule stabilizing agents including taxanes comprising the steps of:
a) determining the level or amount of pStathmin(S38) in at least one sample of said individual and
b) diagnosing or identifying the phenotype and the responsibility and susceptibility to chemotherapeutic treatment based on the amount or level of pStathmin(S38) present in said sample.

6. A method for monitoring the progression of endometrial carcinoma or for determining the transition from low grad to high grade endometrial carcinoma in an individual afflicted with endometrial carcinoma comprising the steps of:
a) determining the level or amount of pStathmin(S38) in a sample of said individual at a first point in time;
b) determining the level or amount of pStathmin(S38) in a sample of said individual at a second point in time; and
c) comparing the level or amount of pStathmin(S38) determined in step a) to the level or amount determined in step b) or to a reference value whereby an increase in the level or the amount relative to a reference value or to the level or amount determined in step a) is indicative for a transition from low grade to high grade or for progression of endometrial carcinoma.

7. A method according to any one of the preceding claims further comprising the step of determining the level or amount of Stathmin.

8. The method according to any one of the preceding claims wherein the sample is selected from tissue, body fluid, in particular, primary and metastatic malignant lesions.

9. The method according to any one of the preceding claims, wherein the level or amount is determined by an immunoassay, in particular, by histochemical or immunofluorescence staining.

10. The method according to any one of the preceding claims wherein high level or amounts of pStathmin(S38), and, optionally, Stathmin, is indicative for high grade aggressive endometrial carcinoma, lower probability of survival and worsened clinical outcome, lower response and susceptibility to chemotherapeutic treatment, in particular, with taxanes, in said individual.

11. The use of a kit for diagnosing or determining the status of endometrial carcinoma, or for predicting the probability of survival, the clinical outcome or determining the treatment course in an individual afflicted or suspected to be afflicted with endometrial carcinoma, or for the stratification of the therapeutic regimen of an individual afflicted with endometrial carcinoma, or for monitoring the progression of endometrial carcinoma or for determining the transition from low grade to high grade endometrial carcinoma in an individual, said kit comprising means for determining the level or amount of pStathmin(S38) and, optionally, Stathmin; and instructions how to use said test kit for a method preferably for a method according to any one of claims 1 to 10.

12. The use of a kit according to claim 11, whereby said kit is an immunohistochemical or immunofluorescence kit.

13. An assay for conducting the method according to any one of claims 1 to 11 comprising means for determining the level or amount of pStathmin(S38) and, optionally, Stathmin.

14. The method according to any one of claims 1 to 10 for differentiation of complex hyperplasia with atypia or endometrial cancer.
